(19) Europäisches Patentamt
European Patent Office
Office européen des brevets

(11) Publication number: **0 049 830**
**B1**

(12) # EUROPEAN PATENT SPECIFICATION

(45) Date of publication of patent specification: **04.09.85**

(51) Int. Cl.$^4$: **A 61 K 7/16**

(21) Application number: **81107848.4**

(22) Date of filing: **02.10.81**

(54) **Mouthwash compositions comprising hexetidine and zinc salts for the synergistic inhibition of dental plaque.**

(30) Priority: **10.10.80 GB 8032743**

(43) Date of publication of application:
**21.04.82 Bulletin 82/16**

(45) Publication of the grant of the patent:
**04.09.85 Bulletin 85/36**

(84) Designated Contracting States:
**AT BE CH DE FR GB IT LI LU NL SE**

(56) References cited:
**CH-A- 337 615**
**DE-A-1 966 450**
**DE-A-2 012 328**
**GB-A-2 084 870**
**US-A-3 095 356**
**US-A-3 989 814**
**US-A-4 022 880**

**Dictionnaire VIDAL, 1982, 58th Ed., pp. 615-616**

(73) Proprietor: **UNILEVER NV**
**Burgemeester s'Jacobplein 1 P.O. Box 760**
**NL-3000 DK Rotterdam (NL)**
(84) **BE CH DE FR IT LI LU NL SE AT**

(73) Proprietor: **UNILEVER PLC**
**Unilever House Blackfriars P O Box 68**
**London EC4P 4BQ (GB)**
(84) **GB**

(72) Inventor: **Mühlemann, Hans R. Prof. Dr.**
**Beustweg 8**
**CH-8032 Zürich (CH)**
Inventor: **Saxer, Peter, Dr. med. dent.**
**Steinbrüchelstrasse 30**
**CH-8053 Zürich (CH)**

(74) Representative: **Doucy, Robert Henry**
**Unilever PLC Patents Division P.O. Box 68**
**Unilever House**
**London EC4P 4BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 049 830 B1**

**Description**

This invention relates to mouthwashes.

Dental plaque is a dense, tenacious deposit of oral bacteria. Calcification (mineralization) of dental plaque results in dental calculus. Calculus formation depends on the presence of plaque.

Plaque forms and grows on the surface of teeth, preferentially in the cervical, interdental and fissure areas and also on dental restorations, crowns, bridges and dentures. Plaque is the obligate cause of dental caries, plaque and calculus are the causes of inflammatory destruction of the tooth-supporting gingival and periodontal structures (periodontitis).

A wide variety of chemical agents have been suggested in the art of retarding plaque formation and the resulting plaque diseases. Mechanical removal of plaque is attempted with oral hygiene measures, but average toothbrushing only partially results in total plaque removal. Therefore, the additional use of chemical antibacterials inhibiting plaque formation in inaccessible dental areas is indicated. Such germicides are phenolic compounds, halogenated bis-phenols (e.g. hexachlorophene), organic mercurials, hydroxyquinolines, iodine esters of hydroxybenzoic acids, chloramine T, and surface active compounds (detergents) among others. These germicides are excellent laboratory disinfectants but relatively poor in vivo plaque inhibitors in contrast to cationic organic antibacterial agents, e.g. water soluble salts of cetylpyridinium, of quaternary ammonium bases (e.g. benzalkonium chloride), of alkylamines (e.g. fluoride of N,N,N'-tris-(2-hydroxyethyl)-N'-octadecyl-1,3-diaminopropane) and of cationic amidines, e.g. water soluble salts of chlorhexidines, such as Hibitane, Alexidine and Vantocil.

The effect on plaque formation of mouthwashes used daily and containing cetylpyridinium chloride (1,2), cetylpyridinium chloride plus a quaternary ammonium base (Domiphen bromide) (3) or benzethonium chloride alone (4) is relatively modest when compared with amidines. This is evident when comparing mouthwashes containing chlorhexidine digluconate with other mouthwash antibacterials (5,6) including hexetidine (7). Unfortunately, mouthrinsing with amidines will rapidly produce a cosmetically unacceptable brown staining of teeth (8). Cationic detergents and amidines have also been found to irritate the oral mucosa even when used at recommended therapeutic concentrations in rinsing solutions (9).

The antiplaque properties of metal ions were mentioned as early as 1940 (10). US Patent 1 593 485 refers to zinc phenolsulfonate as bactericide. The use of zinc oxide or zinc phosphate for the stabilization of dental creams is described in US patent 3 622 662. For the same purpose zinc oxide and zinc sulphate are claimed in US patent 3 624 199. Effervescent antiplaque tablets containing zinc chloride are described in US patents 3 888 976 and 3 772 431. Pader (US patent 4 082 841) reported about the anticalculus effect of zinc ions. A composition reducing the formation of dental calculus is reported in the German patent 2 203 379. Antiplaque compositions based on zinc salts of carboxymethyloxysuccinic acid are described in US patent 4 144 323.

Various zinc salts have been studied in clinical trials to determine their antiplaque potential when use in mouthwashes but the results were conflicting. A zinc chloride solution at 1000 ppm zinc concentration was reported in one study to be effective (11), in another trial totally ineffective (18). Soluble zinc citrate had a small plaque inhibitory action at zinc concentrations of 600 ppm (19) and 1000 ppm (20), respectively. Zinc chloride rinses (Zn=1000 ppm) clearly depressed calculus formation (21), zinc chloride bound to carboxy-sulphate- and phosphate groups was reported to have deodorant properties. Zinc chloride complexed to polymers of carboxyl sulfate and phosphate groups prevents or controls mouth odour (US—A—4,138,477).

In recent years the mechanisms of plaque inhibiting properties of metal ions have become increasingly evident (11, 12, 13, 14). Plaque apparently is only loosely adsorbed on the enamel under the metal ion action. Metal ions also interfere with the metabolic (enzymatic) activity of bacteria, e.g. with acid formation from dietary carbohydrates. Among silver, zinc, magnesium and aluminum, stannous ions seem to possess the highest antiplaque activity (11, 15, 16). Unfortunately, it is difficult to prevent hydrolysis and precipitate formation in mouthwashes containing water-soluble tin salts (17). In addition, stannous fluoride is known to stain teeth (13). Yellowish discoloration does not occur with the use of zinc ions, such as zinc fluoride, zinc acetate and zinc chloride.

A mouthwash containing a 0.22% zinc chloride (Lavoris) has been marketed in the US for many years. There is a general agreement that zinc salts have a beneficial astringent effect on inflammed oral mucosae and that they are modest plaque inhibitors.

Antiplaque and anticalculus effects have been claimed for the combination of zinc ions with tetradecylamine (US patent 4 146 607), with glycine (UK patent application GB 2 052 978A), and with enzymes (US patent 4 082 841). The combination of zinc salts with amidines (European patent application 0 026 252) was reported to prevent tooth discolorations without loss of amidine antiplaque action.

Antiplaque effects of oral rinses containing zinc salts and antibacterials have been reported in US patent 4 022 880. Some scientific investigations showed however possible incompatibilities between solutions of zinc salts and of quaternary ammonium bases (18). In another trial zinc acetate combined with chlorhexidine digluconate was slightly but not significantly superior in the antiplaque action when compared with chlorhexidine alone (20).

US patent 3 989 814 discloses a dentifrice having superior cleaning and polishing characteristics containing an abrasive system including calcium pyrophosphate which has been pre-treated with a

non-toxic zinc compound. Among various optional ingredients which may be incorporated in the dentifrice is mentioned hexetidine.

Plaque and calculus reduction studies with combinations of zinc salts and the water-insoluble pyrimidine base hexetidine have hitherto not been described in the literature.

It is an object of this invention to provide a novel antiplaque mouthwash composition comprising hexetidine in combination with a salt of zinc having a non-polymeric anion.

Hexetidine, a saturated pyrimidine derivate, the analytical profile of which was recently reported (23) has been used in mouthrinses for over 20 years already. The chemical formula is

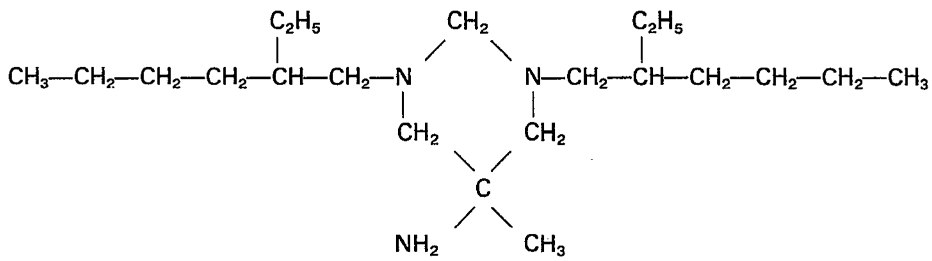

Hexetidine

1,3-bis(2-ethylhexyl)hexahydro-5-methyl-5-pyrimidineamine.

Well known marketed mouthwashes containing 0.1 to 0.2 per cent hexetidine are Drossadine, Hexoral, Hextril, Oraldene, Sterisol. They have been mainly recommended as pre- and postoperative astringent oral antiseptics and for the elimination of unpleasant breath.

Antiseptic oral effects of hexetidine, its depressive action on inflammed oral mucosae and its deodorant effects in the oral cavity were for the first time extensively reported in 1958 at a Symposium on Hexetidine, Northwestern University, Chicago (24, 25).

Similar to zinc salt solutions, the plaque inhibiting action of 0.1 per cent hexetidine alone is also relatively moderate and more than 50 per cent smaller when compared with 0.2 per cent chlorhexidine digluconate rinses (7, 26, 29, 30, 31). In addition, there are indications that 1) retention of hexetidine in the oral cavity after rising, and 2) subsequent antiglycolytic effects in plaque are less pronounced than in the case of chlorhexidine (27) and amine chloride (28), respectively.

It has now been discovered that a salt of zinc having a non-polymeric anion dissolved and emulgated at a physiological pH together with hexetidine in an alcohol containing aqueous mouthwash has a pronounced synergistic antiplaque action in animal caries tests, clinical experiments and microbiological media. Clinical plaque reduction is similar or may exceed the one by chlorhexidine, and without tooth staining (29, 32).

Scope of the invention

The invention encompasses novel mouthwash compositions containing hexetidine in combination with zinc salts and which are stable and used at a physiological oral pH.

The combination of hexetidine with zinc salts results in a highly synergistic statistically significant inhibition of oral microorganisms and of plaque formation on teeth and dental restorations improving the oral hygiene and depressing simultaneously the inflammation of gums (gingivitis).

The use of the zinc-hexetidine-combination at 8 hour intervals, causes almost total inhibition of growth of plaque without irritating the oral mucosae and without staining the teeth.

The antibacterial effect of the zinc-hexetidine-combination is specific. Replacement of zinc by other metal ions or replacement of hexetidine by, for example amidines or quaternary ammonium bases will annihilate the antibacterial synergism of the zinc-hexetidine-combination at low concentrations.

Preferably the composition according to the invention contains simultaneously hexetidine and zinc fluoride or hexetidine, zinc fluoride plus another zinc salt or hexetidine and a zinc salt other than zinc fluoride or hexetidine, zinc fluoride and sodium or potassium fluoride.

Also, according to the invention, preferably the hexetidine is solubilised and emulgated at physiological pH, advantageously in a nonionic emulgator (Cremophor) and ethanol.

Examples of zinc salts for use in mouthwash compositions of this invention are zinc fluoride, zinc citrate and zinc acetate.

Experimental evaluation

Inhibition of plaque formation in

a) animal experiments
b) clinical investigations
c) microbiological media

a) Animal experimentation

When 21 days old, four groups of 13 Osborne-Mendel rats each were inoculated orally with a suspension of Streptococcus mutans OMZ 176 and Actinomyces viscosus Ny 1. Subsequently the cariogenic sucrose containing diet 2000 a was offered ad libitum for 3 weeks. The solutions specified in Table I were applied to the rat molars 3 times per day. At the end of the experiment the extent of plaque formed on the molars was evaluated with a scoring system. The findings are summarized in Table I.

TABLE I

The effects on plaque formation of various solutions containing zinc ions and hexetidine in Osborne-Mendel rats fed the cariogenic diet 2000 a

| Topically applied compounds | Abbreviated designation | Average extent of plaque formed on molars+, PU |
|---|---|---|
| A 250 ppm F as NaF | $H_2O$ Control | 3.5 |
| B 250 ppm F as NaF+ 750 ppm F Hexetidine | Hex 750 ppm | 3.4 |
| C 250 ppm F from $ZnF_2$ 750 ppm Zn from $ZnF_2$ +zinc acetate | Zinc 750 ppm | 2.2 |
| D 250 ppm F from $ZnF_2$ 750 ppm Zn from $ZnF_2$ +zinc acetate 750 ppm Hexetidine | Hex 750 ppm + Zinc 750 ppm | 1.1 |

+ theoretical maximum value=4.0

* $P_F$ significances.

Inhibition (1.1 PU) of plaque formation was synergistic and significantly greatest in the case of zinc fluoride/zinc acetate plus hexetidine, combined in one solution (treatment D). Hexetidine alone was without effect (3.4 PU), zinc fluoride was inhibitory (2.2 PU).

b) Clinical investigations

Six female dental hygienist students, 20 to 26 years old, and known from previous investigations to be heavy plaque formers when consuming sucrose candies frequently, participated in a clinical testing of four mouthwash solutions. Prior to each of the four consecutive 7-day-rinsing periods, the subjects had their teeth cleaned, pumiced and polished professionally. The volunteers then were randomly assigned to one of the 4 antiplaque rinsing treatments listed below. For ethical considerations a neutral water control rinse was omitted. The subjects had to rinse 3 times a day (twice under supervision) for 30 s and had to refrain from tooth brushing and other mechanical hygiene procedures.

List of rinsing solutions (treatments)

1) Solution containing 0.1 per cent chlorhexidine digluconate freshly prepared from Plak Out Liquid. Plak Out Liquid is a 10 per cent chlorhexidine concentrate (HAWE, Gentilino-Lugano, Switzerland), 4 drops in 10 ml water providing a 0.1% solution.

2) Solution containing 0.1 per cent hexetidine plus zinc fluoride ($ZnF_2$) at 215 ppm Zn and 250 ppm F (adapted with sodium fluoride) concentrations.

3) Solution containing N,N,N'-tris-(2-hydroxyethyl)-N'-octadecyl-1,3-diaminopropane dihydrofluoride (amine fluoride, compound 297) at 250 ppm F plus zinc fluoride ($ZnF_2$) at 215 ppm Zn.

4) Solution containing amine fluoride 297 (250 ppm F) plus stannous fluoride ($SnF_2$) at 250 ppm F.

Lycasin 8055 (Roquette Frères, Lestrem, France) was added to all treatments. It is a sweetening agent containing mainly maltitol, sorbitol and hydrogenated dextrines.

Each subject rinsed with the 4 experimental solutions allocated at random. There were no time intervals between the treatments. The study was performed double blind.

The rate of plaque formation was documented at the end of each experimental period with standardized color photography of the teeth stained with Displac. The extent of plaque formation was determined planimetrically using projected Kodachrome slides.

The findings are summarized in Table II.

TABLE II

The effect of mouthwash solutions containing chlorhexidine, hexetidine+zinc fluoride ($ZnF_2$), amine fluoride 297∮+$ZnF_2$, amine fluoride 297+$SnF_2$, respectively on plaque formation in 6 subjects abstaining from mechanical oral hygiene measures during 4 consecutive 7-day-rinsing periods. Averages of planimetric units PU±standard deviation

| Rinsing solution | Positive control, chlorhexidine | Hexetidine +$ZnF_2$ | Amine fluoride 297∮+$ZnF_2$ | Amine fluoride 297∮+$SnF_2$ |
|---|---|---|---|---|
| 7-day extent of plaque, PU | 188±90 | 30±9 | 101±54 | 61±22 |
| Theoretical amount of plaque formed per day | 26.9 | 4.3 | 14.4 | 8.7 |

∮ N,N,N'-tris-(2-hydroxyethyl)-N'-octadecyl-1,3-diaminopropane. 2HF.

Inhibition of plaque formation with the hexetidine-$ZnF_2$-combination was significantly superior to the chlorhexidine rinse ($P_t<0.01$), and to the rinses, not part of this invention, containing amine fluoride+$SnF_2$ ($P_t<0.05$) and amine fluoride+$ZnF_2$ ($P_t<0.05$).

When rinsing with 0.1 per cent chlorhexidine an average of 26.9 PU per day were formed (Table II). The same volunteers who in a previous and similar investigation rinsed with water containing Lycasin 8055 had produced an average of 50.2 PU per day suggesting therefore relatively strong antiplaque activity of the 0.1 per cent chlorhexidine rinse (Plak Out) in the present study (26.9 PU/day).

Staining of teeth by the treatment with hexetidine+$ZnF_2$ did not occur. A yellowish stain, however, was produced by the chlorhexidine rinse.

All the rinsing solutions showed stability and remained clear over the treatment periods and several months thereafter.

In a second clinical test with 10 dental hygiene students the antiplaque effect of zinc fluoride, hexetidine, $ZnF_2$+hexetidine was tested. The rinsing solutions were:

1) Control (NaF, 250 ppm F)
2) $ZnF_2$ (250 ppm F, 750 ppm Zn)
3) Hexetidine (750 ppm)+NaF (250 ppm)
4) $ZnF_2$ (250 ppm F, 750 ppm Zn) plus hexetidine (750 ppm) pH 5.9.

To the solutions were added aromatics. They were well tasting and non-irritative to oral mucosae. The experimental procedures were similar to those reported for the previous experiment. At the beginning of the 4 one-week-rinsing periods the 10 female dental hygiene students were given a professional tooth cleaning. They then refrained from mechanical oral hygiene procedures and rinsed 3 times per day (twice under supervision) with 10 ml for 30 s. The test solutions were assigned at random. The plaque reductions observed in the double blind study are summarized in Table III.

TABLE III

Average plaque indices±standard deviations and average planimetric units in 10 volunteers refraining from tooth brushing and other mechanical oral hygiene procedures but rinsing during four one-week test periods with hexetidine- and zinc fluoride solutions

| | Pl I Plaque index (Silness-Loe) | Percentage of vestibular tooth surfaces covered with plaque (PU) |
|---|---|---|
| Control | 2.58±0.28 | 33.4±16.57 |
| $ZnF_2$ | 2.16±0.38 | 23.2±14.44 |
| Hexetidine | 2.11±0.72 | 17.4±8.07 |
| $ZnF_2$+Hexetidine | 0.77±0.55 | 3.5±2.17 |

The results show the synergistic and highly significant plaque inhibition by the zinc-hexetidine-mouthwash.

c) Microbiological investigation

The synergistic antibacterial effect of the zinc-hexetidine-combination is clearly evident in microbiological investigations. 24 h-growth and acid production of Streptococcus mutans cultured in glucose-containing synthetic FMC-broth are totally inhibited by zinc acetate alone at a minimal concentration of 40 ppm Zn, and by hexetidine alone at 0.4 ppm. The combination of both agents in the broth results in total bacterial inhibition at a concentration of 4.0 ppm Zn and 0.08 ppm hexetidine (33). Zinc or hexetidine are without antibacterial effects at these low concentrations.

The synergistic antibacterial effect can also be observed if zinc and hexetidine, respectively, are used sequentially on washed streptococcal cultures, zinc first and subsequently hexetidine and vice versa.

The synergistic inhibition of plaque microorganisms with the zinc-hexetidine-combination is specific. Other metal ions, e.g. stannous ions combined with hexetidine or other antibacterials combined with zinc only cause additive antibacterial effects.

The combination of zinc with antibacterials has been described in US patent 4 022 880. Claims are only made for additive effects: "our discovery concerns the co-action of zinc ions and an antibacterial agent, rather than a well defined synergistic property" (column 4 of US 4 022 880). The antibacterials listed in US patent 4 022 880 include amidines, quaternary ammonium bases, phenolics but not pyrimidine amine bases nor hexetidine specifically. In addition many zinc salts are referred to, but not zinc fluoride.

Summary of experimental evaluations

The synergistic and specific antiplaque effect of the zinc-hexetidine combination has been shown in a rat caries test and in two independent clinical plaque studies, using organoleptically acceptable mouth rinses. Synergistic inhibition was also observed in microbiological laboratory investigations.

Examples of mouthwash compositions

Components of exemplary mouthwash compositions in accordance with the present invention are given in the following examples:

Example I

| | |
|---|---|
| Hexetidine | 0.1 |
| Zinc fluoride | 0.1 |
| Sodium fluoride | 0.05 |
| Glycerol | 5.0 |
| Cremophor EL | 0.25 |
| Ethanol | 10.0 |
| Polyoxyethylen-sorbitanmonoleate | 0.1 |
| Aromatics, saccharin | 0.15 |
| Water | ad 100.0 |

Example II

| | |
|---|---|
| Hexetidine | 0.2 |
| Zinc citrate | 0.15 |
| Glycerol | 5.0 |
| Cremophor EL | 0.25 |
| Ethanol | 10.0 |
| Aromatics, saccharin | 0.1 |
| Water | ad 100.0 |

The names Hibitane, Alexidine, Vantocil, Domiphen bromide, Lavoris, Drossadine, Hexoral, Hextril, Oraldene, Sterisol, Lycasin, Cremophor, Plak Out, Displac and Kodachrome are trade marks.

Literature references

1. Carter, H. G., Barnes, G. P.: Effects of three mouthwashes on existing dental plaque accumulations. J Prev Dent 2, 10, 1975.

2. Ciancio, S. G., Mather, M. L., Bunnell, H. L.: Effect of a quaternary ammonium type mouthrinse on formed plaque. J Dent Res 54 A, Abstract 585, 1975.

3. Barnes, G. P., Roberts, D. W. Katz, R. V., Woolridge, E. D.: Effects of two cetylpyridinium chloride-containing mouthwashes on bacterial plaque. J Periodontol 47, 419, 1976.

4. Volpe, A. R., Kupczak, L. J., Brant, J. H., King, W. J., Kestenbaum, R. C., Schlissel, H. J.: Antimicrobial control of bacterial plaque and calculus and the effects of these agents on oral flora. J Dent Res 48, 832, 1969.

5. Gjermo, P., Baastad, K. L., Rölla, G.: The plaque inhibiting capacity of 11 antibacterial compounds. J Periodont Res 5, 102, 1970.

6. Mühlemann, H. R., Hülss, D., Steiner, E.: Antimicrobial rinses and proximal plaque on removable gold crowns. Helv Odont Acta 17, 89, 1973.

7. Bergenholtz, A., Hänström, L.: The plaque-inhibiting effect of hexetidine (Oraldene®)-mouthwash compared to that of chlorhexidine. Community Dent Oral Epidemiol 2, 70, 1974.

8. Flötra, L., Gjermo, P., Rölla, G., Waerhaug, J.: Side effects of chlorhexidine mouthwashes. Scand J Dent Res 79, 119, 1971.

9. Flötra, L.: Different modes of chlorhexidine application and related local side effects. J Periodont Res 8, Supp. 12, 41, 1973.

10. Hanke, M. T.: Studies on the local factors in dental caries, I. Destruction of plaque and retardation of bacterial growth in the oral cavity. JADA 27, 379, 1940.

11. Skjörland, K., Gjermo, P., Rölla, G.: Effect of some polyvalent cations on plaque formation in vivo. Scand J Dent Res 86, 103, 1978.

12. White, S. T., Taylor, P. P.: The effect of stannous fluoride on plaque scores. J Dent Res 58, 1850, 1979.

13. Svatun, B., Gjermo, P., Eriksen, H. M., Rölla, G.: A comparison of the plaque-inhibiting effect of stannous fluoride and chlorhexidine. Acta Odontol Scand 35, 247, 1977.

14. Hock, J., Tinanoff, N.: Resolution of gingivitis in dogs following topical applications of 0.4% stannous fluoride and toothbrushing. J Dent Res 58, 1652, 1979.

15. Svatun, B.: Plaque-inhibiting effect of dentifrices containing stannous fluoride. Acta Odontol Scand 36, 205, 1978.

16. Gjermo, P., Rölla, G.: Plaque inhibition by antibacterial dentifrices. Scand J Dent Res 78, 464, 1970.

17. Shannon, I. L. and Gibson, W. A.: Shelf life of aqueous solutions of stannous fluoride. USA School of Aerospace Medicine, Brooks Air Force Base, Texas. SAM-TDR-63-104.

18. Compton, F. H., Beagrie, G. S.: Inhibitory effect of benzethonium and zinc chloride mouthrinses on human dental plaque and gingivitis. J Clin Periodontol 2, 33, 1975.

19. Addy, M., Richards, J., Williams, G.: Effects of a zinc citrate mouthwash on dental plaque and salivary bacteria. J Clin Periodontol 7, 309, 1980.

20. Waler, S. M., Rölla, G.: Plaque inhibiting effect of combinations of chlorhexidine and the metal ions zinc and tin. A preliminary report. Acta Odontol Scand 38, 201, 1980.

21. Schmid, M. O., Schait, A., Mühlemann, H. R.: Effect of a zinc chloride mouthrinse on calculous deposits formed on foils. Helv Odont Acta 18, 22, 1974.

22. Picozzi, A., Fischman, S. T., Pader, M., Cancro, L. P.: Calculus inhibition in humans. J Periodontol 43, 692, 1972.

23. Satzinger, G., Herrmann, W., Zimmermann, F.: Analytisches Profil des Rein-Hexetidins. Drug Research 25, 1849, 1975. (Gödecke AG, Freiburg i. Br., West Germany).

24. Gödecke Publication report on Hexoral®.

25. Report of Warner-Chilcott on Hextril®.

26. Mühlemann, H. R., Hülss, D., Steiner, E.: Antimicrobial rinses and proximal plaque on removable gold crowns. Helv Odont Acta 17, 89, 1973.

27. Hefti, A., Widmer, B.: Reduktion des Keimpegels in der Mundhöhle vor zahnärztlichen Behandlungen durch Mundwässer und Mundantiseptika. Schweiz Mschr Zahnheilk 90, 73, 1980.

28. Breitenmoser, Th.: The Antiglycolytic action on dental plaque of amine chloride. Helv Odont Acta 19, 13, 1975.

29. Mühlemann, H. R.: Auf dem Weg zum sauberen Zahn? SWISS DENT 2, No. 1—2, 7, 1981.

30. Mühlemann, H. R., Saxer, U. P.: Plaque inhibition by rinsing solutions containing aminefluoride, hexetidine and zinc ions. Preprinted Abstract No. 17, ORCA-Congress 1981, Erfurt, DDR.

31. Saxer, U. P., Mühlemann, H. R.: Plaque-inhibiting effect of zinc ions and fluoride, hexetidine and their combinations. Preprinted Abstract No. 18, ORCA-Congress 1981, Erfurt, DDR.

32. Saxer, U. P.: Kommt die chemische Zahnbürste? SWISS DENT 1, No. 12, 24, 1980.

33. Mörmann, J. E., Mühlemann, H. R.: Synergistic in vitro inhibition of S. mutans by Zn and hexetidine. Abstract accepted for IADR-Congress 1981, Groningen, Netherlands.

**Claims**

1. An antiplaque mouthwash composition comprising hexetidine in combination with a salt of zinc having a non-polymeric anion.

2. A composition as claimed in Claim 1, wherein the zinc salt is zinc fluoride.

3. A composition as claimed in Claim 2, wherein the composition also comprises sodium fluoride or potassium fluoride.

4. A composition as claimed in Claim 2, wherein the composition also comprises another zinc salt.

5. A composition as claimed in Claim 1, wherein the zinc salt is a salt other than zinc fluoride.

6. A composition as claimed in Claim 5, wherein the zinc salt is zinc citrate.

7. A composition as claimed in any of the preceding claims, wherein the hexetidine and zinc salt are solubilised and emulgated.

**Patentansprüche**

1. Antiplaque-Mundwasserzusammensetzung, umfassend Hexetidin in Kombination mit einem Zinksalz mit nicht-polymerem Anion.

2. Zusammensetzung nach Anspruch 1, worin das Zinksalz Zinkfluorid ist.

3. Zusammensetzung nach Anspruch 2, worin die Zusammensetzung auch Natriumfluorid oder Kaliumfluorid umfaßt.

4. Zusammensetzung nach Anspruch 2, worin die Zusammensetzung auch ein weiteres Zinksalz umfaßt.

5. Zusammensetzung nach Anspruch 1, worin das Zinksalz ein anderes Salz als Zinkfluorid ist.

6. Zusammensetzung nach Anspruch 5, worin das Zinksalz Zinkcitrat ist.

7. Zusammensetzung nach irgend einem der vorhergehenden Ansprüche, worin das Hexetidin und Zinksalz löslich gemacht und emulgiert sind.

**Revendications**

1. Une composition d'eau dentifrice antiplaque comprenant de l'hexétidine en combinaison avec un sel de zinc ayant un anion non polymère.

2. Une composition comme revendiqué dans la revendication 1, dans laquelle le sel de zinc est le fluorure de zinc.

3. Une composition comme revendiqué dans la revendication 2, dans laquelle la composition contient également du fluorure de sodium ou du fluorure de potassium.

4. Une composition comme revendiqué dans la revendication 2, dans laquelle la composition comprend également un autre sel de zinc.

5. Une composition comme revendiqué dans la revendication 1, dans laquelle le sel de zinc est un sel autre que le fluorure de zinc.

6. Une composition comme revendiqué dans la revendication 5, dans laquelle le sel de zinc est le citrate de zinc.

7. Une composition comme revendiqué dans l'une quelconque des revendications précédentes dans laquelle l'hexétidine et le sel de zinc sont solubilisés et émulsifiés.